# EUROPEAN PATENT SPECIFICATION

(11) **EP 0 695 750 B1**
(45) Date of publication and mention of the grant of the patent: **01.03.2000**
(21) Application number: 95112150.8
(22) Date of filing: 02.08.1995
(51) Int. Cl.: C07D 471/04, G01N 33/68

(54) **Naphthyridinium derivative, method for its production; antibody against said derivative and the use of said antibody**
Naphthyridinium-Derivat, Verfahren zu seiner Herstellung, Antikörper gegen dieses Derivat und die Verwendung dieses Antikörpers
Dérivé de naphtyridinium, procédé pour sa préparation, anticorps contre ce dérivé et l'application du dit anticorps

(30) Priority: 05.08.1994 JP 20451794
(43) Date of publication of application: 07.02.1996
(73) Proprietor: NIPPON ZOKI PHARMACEUTICAL CO., LTD., Chuo-ku, Osaka (JP)
(72) Inventor: Horiuchi, Seikoh, Kumamoto-shi, Kumamoto (JP); Ienaga, Kazuharu c/o Inst. of Bioact. Sc. Nippon, Yashiro-cho, Katoh-gun, Hyogo (JP); Nakamura, Ko, c/o Inst. of Bio-Active Sc. Nippon, Yashiro-cho, Katoh-gun, Hyogo (JP)
(74) Representative: Hansen, Bernd, Dr. Dipl.-Chem.

(56) References cited:
- EP-A- 0 563 797
- CHEMICAL ABSTRACTS, vol. 105, no. 21, 24 November 1986 Columbus, Ohio, US; abstract no. 191055r, page 727;
- Min-Xin Fu et al., Diabetes, 41, Suppl. 2, 42-48, 1992
- J. W. Baynes, Diabetes, 40, 405-12, 1991
- T. Miyata et al., Kidney International, 51 (1997), 1170-1181
- S. K. Grandhee et al., Journal of Biological Chemistry, 1991, no. 18, 11649-53
- Min-Xin Fu et al., Journal of Biological Chemistry, 1996, 271 (17), 9982-6

## Description

The present invention relates to a novel naphthyridinium derivative; method for the production of said derivative; antibody prepared using said derivative as a hapten; a method for diagnosis of diabetes, diabetic complications, dialysis-related complications, aging, diseases accompanied by aging, etc. using said derivative or antibody thereof; and a method for evaluation of effect of pharmaceuticals which are effective for such diabetes-related diseases, aging and diseases accompanied by aging.

In 1968, Glycosylhemoglobin (HbAlc) which is one of the minor components of hemoglobin was identified in vivo and was found to increase in diabetes patients (S. Rahbar, Clin. Clim. Acta, 22, 296, (1968)). Since then, the biological meaning of the Maillard reaction and, particularly, the relation between aging and diabetes have been receiving public attentions.

The Maillard reaction may be classified into a first stage, wherein a Schiff base is formed from an amino group in protein and an aldehyde group in a reduced sugar and is stabilized as a result of Amadori rearrangement, and a latter stage, wherein the above is transferred, after a long series of reactions, to Advanced Glycation End Product (AGE) which is characterized by fluorescence, color change to brown and molecular crosslinking. The fluorescence which is known as one of the characteristic changes in the AGE is significantly higher in diabetic patients than healthy and ordinary persons and is suggested to have a correlation with the onset of diabetic complications such as diabetic nephrosis, arteriosclerosis, nervous disturbance, retinal diseases, cataract, etc. In addtion, since fluorescence of proteins accumulating in the serum increases during dialysis, the relation between AGE and dialysis-related amyloidosis is suggested. The basic structure of AGE has been analysed, however, many obscure matters are still remaining at present.

Meanwhile, as a result of many studies, it has been clarified that diabetic patients are subject to a higher oxygen-stressed condition than normal persons. It is suggested that said condition is caused by free radicals produced during glycation of proteins and automatic oxidation of glucose. The free radicals also injure the tissues and may become a factor causing diabetic complications and aging. The present inventors have conducted continued studies on the crosslinked substances, as an indicator to diagnose diabetic complications and aging, which are produced by both reactions of nonenzymatic glycation and oxidation of proteins. As a result thereof, they found novel naphthyridinium derivatives which have different fluorescent characteristics from that of known AGE whereby the present invention has been accomplished.

An object of the present invention is to offer a novel naphthyridinium derivative; method for the production of said derivative; antibody prepared using said derivative as a hapten; a method for diagnosis of diabetes, diabetic complications, dialysis-related complications, aging, diseases accompanied by aging, etc. using said derivative or antigen thereof; and a method for evaluation of effect of pharmaceuticals for diabetes-related diseases, aging, and accompanying diseases.

A novel naphthyridinium derivative of the present invention is the substance represented by the following general formula (I), 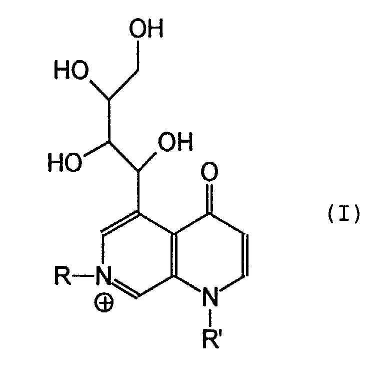 in which R and R' are alkyl which may be the same or different and which may be substituted with one or more amino, protected amino and/or carboxyl group(s), as well as salts thereof.

Preferred alkyl for R and R' in the above general formula (I) are linear or branched alkyl having 1 to 6 carbon atom(s) such as methyl, ethyl, propyl, isopropyl, butyl, isobutyl, sec-butyl, tert-butyl, pentyl, isopentyl, neopentyl, tert-pentyl and hexyl. The alkyl groups also include cycloalkyl groups.

Said alkyl may be substituted with one or more amino, protected amino and/or carboxyl group(s). With regard to the protective groups for the amino group, those which are commonly used in the field of peptide synthesis, may be utilized. They are, for example, formyl, acetyl, tosyl, benzyloxycarbonyl, p-methoxybenzyloxycarbonyl, p-chlorobenzyloxycarbonyl, p-nitrobenzyloxycarbonyl, p-phenylazobenzyloxycarbonyl, p-methoxyphenylazobenzyloxycarbonyl, tert-butoxycarbonyl, tert-amyloxycarbonyl, p-biphenylisopropyloxycarbonyl and diisopropylmethyloxycarbonyl.

Among the substances of the present invention represented by the general formula (I), naphthyridinium derivatives wherein R and R' are alkyl having amino and/or carboxyl can be easily combined with carrier protein as haptens and are useful particularly for preparation of the antibody of the present invention. As to a carrier for combining with a hapten for preparation of antibody of this invention, the commonly-used carriers such as protein (e.g. serum albumin, keyhole lympet hemocyanin) and polymers (e.g. polylysine) may be used.

The naphthyridinium derivatives of the present invention include salts of the substances represented by the general formula (I). They are, for example, salts with alkali metal such as sodium, potassium and lithium; alkali earth metal such as magnesium, calcium and barium; and other metals such as aluminum. Furthermore, salts with halogens, such as fluorine, chlorine, bromine and iodine and salts with sulfate, nitrate, phosphate, perhydrochlorate, thiocyanate, borate, formate, acetate, haloacetate, propionate, glycolate, citrate, tartarate, succinate, gluconate, lactate, malonate, fumarate, anthranilate, benzoate, cinnamate, p-toluenesulfonate, naphtalenesulfonate, sulfanilate and trifluoroacetate and the like are included. Further examples are addition salts with acids such as hydrochloric acid, sulfuric acid, nitric acid, hydrobromic acid, phosphoric acid, perhydrochloric acid, thiocyanic acid, boric acid, formic acid, acetic acid, haloacetic acid, propionic acid, glycolic acid, citric acid, tartaric acid, succinic acid, gluconic acid, lactic acid, malonic acid, fumaric acid, anthranilic acid, benzoic acid, cinnamic acid, p-toluenesulfonic acid, naphtalenesulfonic acid, sulfanilic acid and trifluoroacetic acid; and salts with ammonia or with organic bases such as organic amines.

Those salts can be manufactured by conventional methods from the naphthyridinium derivatives of the present invention in free form and vice versa.

When the substances of the present invention have stereoisomers such as cis-trans isomers, optical isomers, conformational isomers, etc., the present invention includes any of such stereoisomers.

Methods for the production of the substances of the present invention are given in the Examples.

Thus, a substance of formula R-NH₂ or R'-NH₂ (amine component; wherein R and R' are the same groups as mentioned already) is mixed with sugars, for example, hexose such as glucose, fructose, galactose, mannose or deoxyglucose, heptose such as sedoheptulose, amino sugar such as glycosamine or galactosamine, oligosaccharide such as saccharose, to give the substance of the present invention.

There is no particular restriction for the reaction conditions such as reaction temperature, reaction time, pH, etc. but they may be properly established. A procedure is to allow the reaction mixture to stand at room temperature, however, the reaction can be accelerated by heating and the like.

The substances of the present invention prepared as such were purified by common means such as distillation, chromatography, recrystallization, etc. and identified by means of NMR, mass analysis, fluorescence spectrum, etc.

### Example 1.

28.2 g of α-N-acetyl-L-lysine and 27 g of glucose were dissolved in 750 ml of 250 mM phosphate buffer (pH: 7.4) and allowed to stand at 37°C for 8 weeks. The reaction solution was added to an ion-exchange resin of sulfonate type/DIAION PK-216 H-form (Mitsubishi Kasei Corp.) and then the column was well washed with water. The fraction eluted by 2N aqueous ammonia was concentrated to dryness. This was further separated by DEVELOSIL ODS LOP-45S column (Nomura Chemical Corp.). The methanol content in the elution solvent is increased stepwise, for example, in the order of 0.2% trifluoro acetic acid (TFA), 10% methanol/0.2% TFA and 20% methanol/0.2% TFA. The fraction eluted by the solvent containing 20% methanol was purified by reversed phase HPLC/STR ODS-II column (Shimadzu Techno-Research Inc.) to give 1,7-bis [6-(N-acetyl-L-norleucyl)]-5-(1,2,3,4-tetrahydroxybutyl)-1,4-dihydro-4-oxo-1,7-naphthyridinium (TFA salt form).
¹³C-NMR (100 MHz, D20): 177.1(s), 177.1(s), 175.8(s), 175.6(s), 149.5(s), 143.8(s),137.9(d), 137.6(d), 122.7(s), 120.2(s), 110.0(d), 93.0(d), 74.0(d), 71.6(d), 66.6(d), 64.6(d), 57.6(t), 54.1(d), 54.1(d), 48.4(t), 32.0(t), 31.7(t), 30.1(t), 30.1(t), 23.8(t), 23.2(t), 23.2(q), 23.2(q)
¹H-NMR (400 MHz, D20): 8.35(1H,d,J=7Hz),7.97(1H,d,J=7Hz), 7.95(1H,s), 6.77(1H,s), 5.46(1H,s), 4.65(1H,m), 4.55(1H,m), 4.54(1H,d,J=9Hz), 4.37(2H,t,J=6Hz), 4.37(1H,m), 4.25(1H,m), 4.0-4,1(2H,m), 3.71(1H,dd,J=7,12Hz), ), 2.02(3H,s), 1.96(3H,s), 1.25-2.1 (6H,m)
Mass analysis (SIMS, Glycerol): M+ (m/z)=609

### Example 2.

41.2 g of γ-aminobutyric acid and 72 g of glucose were dissolved in 1000 ml of 250 mM phosphate buffer (pH: 7.4) and allowed to stand at 37°C for 4 weeks. The reaction solution was added to XAD-2 column (Organo Corp.), and each fraction (80 ml) was collected and analysed by reversed phase HPLC/STR ODS-II column. The fraction containing the objective substances was concentrated, and then further separated by reversed phase column chromatography/YMC-GEL ODS-AM 120 S50 column (YMC Co., Ltd.). The obtained fractions were analysed by the said reversed phase HPLC. The fractions containing objective substance were collected and concentrated, and then purified by removing excess of TFA to give 1,7-bis (3-carboxypropyl)-5-(1,2,3,4-tetrahydroxybutyl)-1,4-dihydro-4-oxo-1,7-naphthyridinium (TFA salt form).
¹³C-NMR (100 MHz, D2O): 180.2(s), 180.2(s),149.3(s), 143.9(s), 137.9(d), 137.8(d), 122.8(s), 120.1(s), 110.0(d), 92.9(d), 74.0(d), 71.5(d), 66.5(d), 64.6(t), 57.2(t), 48.1(t), 34.0(t), 31.5(t), 28.9(t), 27.1(t)
¹H-NMR (400 MHz, D2O): 8.37(1H,d,J=7Hz),7.97(1H,d,J=7Hz), 7.96(1H,s), 6.81(1H,s), 5.45(1H,s), 4.70(1H,m), 4.52(1H,m), 4.52(1H,d,J=9Hz), 4.39(2H,t,J=7Hz), 3.99(1H,d,J=12Hz), 3.98(1H,dd,J=7,9Hz), 3.69(1H,br.dd,J=7,12Hz), 2.15-2.55(8H,m)
Mass analysis (SIMS, Glycerol): M+ (m/z)=439
α-N-tosyl-L-lysine was used as an amine component, the mixing reaction with glucose was carried out in the same manner as mentioned above, the separation and purification were also done to give 1,7-bis[6-(N-tosyl-L-norleucyl)]-5-(1,2,3,4-tetrahydroxybutyl)-1,4-dihydro-4-oxo-1,7-naphthyridinium.
¹H-NMR (400 MHz, D2O/CD3OD): 8.40(1H,d,J=7Hz), 8.00(1H,d,J=7Hz), 7.98(1H,s), 7.66(2H,d,J=8Hz), 7.63(2H,d,J=8Hz), 7.35(2Hd,J=8Hz), 7.34(2H,d,J=8Hz), 6.73(1H,s), 5.41(1H,s), 4.65(1H,m), 4.52(1H,m), 4.52(1H,d,J=9Hz), 4.38(2H,t,J=7Hz), 3.99(1H,br.d,J=12Hz), 3.98(1H,dd,J=7,9Hz), 3.70(1H,m), 3.69(1H,br.dd,J=7,12Hz), 3.64(1H,m), 2.40(3H,s), 2.40(3H,s), 1.3-2.0(12H,m)

### Example 3.

25 g of α-N-tert-butoxycarbonyl-ε-N-benzyloxycarbonyl-L-lysine was dissolved in 300 ml of methanol and reduced by palladium-carbon as a catalyst in the atmosphere of hydrogen to remove benzyloxycarbonyl group. 16.2 g of the resulting α-N-tert-butoxycarbonyl-L-lysine and 12.1 g of glucose were dissolved in
335 ml of 250mM phosphate buffer (pH: 7.3) and allowed to stand at 37°C for 24 days. After the reaction mixture was separated and purified by YMC-GEL ODS-AM 120 S50 column and STR ODS-II column, this was dissolved in TFA and allowed to stand for 30 minutes to remove tert-butoxycarbonyl group to give 1,7-bis[6-(L-norleucyl)]-5-(1,2,3,4-tetrahydroxybutyl)-1,4-dihydro-4-oxo-1,7-naphthyridinium (TFA salt form).

The antibody to the naphthyridinium derivative of the present invention was prepared by a usual method, i.e., the substance combined with keyhole lympet hemocyanin was administered to immunize a rabbit. The obtained antibody significantly reacted with various glycated proteins, however, did not react with normal proteins which were not glycated.

Additionally, it has been clear that the antibody reacts with extracts of human lens and the reaction increases according to aging. The substance of this invention shows immune cross-reaction with antibody which is prepared by using AGE-proteins as antigens.

Several candidates as Advanced Glycation Endproduct (AGE) have been shown and the concerned studies are continued. One of the candidates, a novel pyridinium derivative, was disclosed in EP-A-563,797. The publication showed that the excitation wavelengths of the pyridinium derivatives are from 370 nm to 380 nm. On the other hand, the substances of the present invention have different fluorescent characteristics from that of known AGE. Since the excitation wavelengths of the substances of this invention are about 325 nm, the instant substances cannot be detected by fluoresent analysis using the said excitation wavelengths as reported above. The oxidation reaction is not needed for the production of the said pyridinium derivatives, however, the substances of the present invention should be produced by both reactions of glycation and oxidation from consideration of the characteristic structure. Therefore, the instant substances are much focused as a novel candidate of AGE.

As mentioned above, since many studies clarified that diabetic patients are subject to a higher oxygen-stressed condition than normal persons, the crosslinked substances produced by both reactions of glycation and oxidation in the present invention are highly useful as a new indicator to diagnose diabetic complications and aging.

Accordingly, it is possible to conduct diagnosis of diabetes, diabetic complications (e.g. diabetic nephrosis, diabetic arteriosclerosis, diabetic nervous disturbance, diabetic cataract, diabetic retinal diseases, diabetic minute blood vessel disturbance, etc.), dialysis-related complications, aging and accompanying diseases using the compound of the present invention as an indicator. Moreover, it is possible to conduct evaluation of the pharmaceutical effect with in vitro and in vivo test systems by using the compound of the present invention as an indicator. In addition, the antibody prepared using the compound of the present invention as a hapten can be utilized immunochemically and immunohistochemically in the above-mentioned diagnosis and evaluation.

## Claims

1. A naphthyridinium derivative represented by the general formula (I), 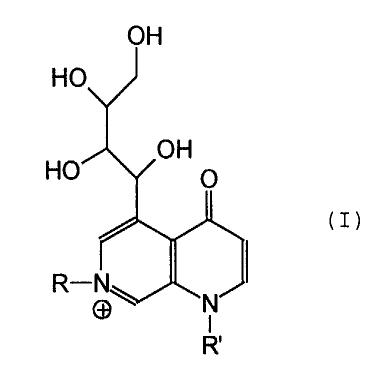 in which R and R' are alkyl having 1 to 6 carbon atom(s) which may be the same or different and which may be substituted with one or more amino, carboxyl and/or protected amino group(s) wherein the protected group is formyl, acetyl, tosyl, benzyloxycarbonyl, p-methoxybenzyloxycarbonyl, p-chlorobenzyloxycarbonyl, p-nitrobenzyloxycarbonyl, p-phenylazobenzyloxycarbonyl, p-methoxyphenylazobenzyloxycarbonyl, tert-butoxycarbonyl, tert-amyloxycarbonyl, p-biphenylisopropyloxycarbonyl and diisopropylmethyloxycarbonyl, as well as salts thereof.

2. Antibody obtainable using a naphthyridinium derivative according to claim 1 as a hapten.

3. Use of a naphthyridinium derivative according to claim 1 as a hapten for the production of the antibody according to claim 2.

4. An in vitro method for diagnosis of diabetes, diabetic complications, dialysis-related complications, aging and diseases accompanied by aging using a naphthyridinium derivative according to claim 1 as an indicator.

5. An vitro method for diagnosis of diabetes, diabetic complications, dialysis-related complications, aging and diseases accompanied by aging using an antibody according to claim 2.

6. An in vitro method for evaluation of effect of pharmaceuticals such as therapeutic agents for diabetes, those for diabetic complications, those for dialysis-related complications, preventive agents for aging and therapeutic agents for diseases accompanied by aging using a naphthyridinium derivative according to claim 1 as an indicator.

7. An in vitro method for evaluation of effect of pharmaceuticals such as therapeutic agents for diabetes, those for diabetic complications, those for dialysis-related complications, preventive agents for aging and therapeutic agents for diseases accompanied by aging using an antibody according to claim 2.

8. Use of a naphthyridinium derivative according to claim 1 for the production of a preparation useful for the diagnosis of diabetes, diabetic complications, dialysis-related complications, aging and diseases accompanied by aging.

9. Use of an antibody according to claim 2 for the production of a preparation useful for the diagnosis of diabetes, diabetic complications, dialysis-related complications, aging and diseases accompanied by aging.

10. Use of a naphthyridinium derivative according to claim 1 for the production of a preparation useful for the evaluation of effect of pharmaceuticals such as therapeutic agents for diabetes, those for diabetic complications, those for dialysis-related complications, preventive agents for aging and therapeutic agents for diseases accompanied by aging.

11. Use of an antibody according to claim 2 for the production of a preparation useful for the evaluation of effect of pharmaceuticals such as therapeutic agents for diabetes, those for diabetic complications, those for dialysis-related complications, preventive agents for aging and therapeutic agents for diseases accompanied by aging.

## Patentansprüche

1. Naphthyridiumderivat der allgemeinen Formel (I): 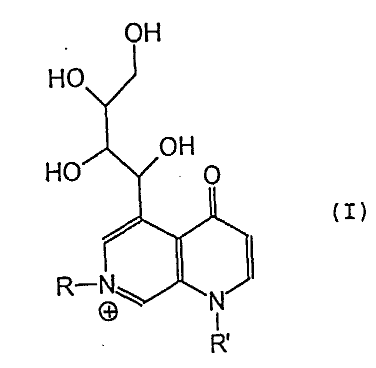 worin R und R' Alkylgruppen mit 1 bis 6 Kohlenstoffatomen sind, die gleich oder verschieden und mit einer oder mehreren Amino-, Carboxyl- und/oder geschützten Aminogruppen sustituiert sein können, worin die Schutzgruppe eine Formyl-, Acetyl-, Tosyl-, Benzyloxycarbonyl-, p-Methoxybenzyloxycarbonyl-, p-Chlorbenzyloxacarbonyl, p-Nitrobenzyloxycarbonyl-, p-Phenylazobenzyloxycarbonyl-, p-Methoxyphenylazobenzyloxycarbonyl-, t-Butoxycarbonyl-, t-Amyloxycarbonyl-, p-Biphenylisopropyloxycarbonyl- und eine Diisopropylmethyloxycarbonylgruppe ist, sowie Salze davon.

2. Antikörper, erhältlich unter Verwendung eines Naphthyridiumderivats gemäß Anspruch 1 als Hapten.

3. Verwendung eines Naphthyridiumderivats gemäß Anspruch 1 als Hapten zur Herstellung des Antikörpers gemäß Anspruch 2.

4. In vitro-Verfahren zur Diagnose von Diabetes, diabetischen Komplikationen, Dialyse-bezogenen Komplikationen, entsprechender Alterungsvorgänge und damit einhergehender Krankheiten, wobei man ein Naphthyridiumderivat gemäß Anspruch 1 als Indikator verwendet.

5. In vitro-Verfahren zur Diagnose von Diabetes, diabetischen Komplikationen, Dialyse-bezogenen Komplikationen, entsprechender Alterungsvorgänge und damit einhergehender Krankheiten, wobei man einen Antikörper gemäß Anspruch 2 verwendet.

6. In vitro-Verfahren zur Bewertung der Wirkung von Pharmazeutika wie therapeutischer Mittel gegen Diabetes, solcher gegen diabetische Komplikationen, solcher gegen Dialyse-bezogene Komplikationen, vorbeugender Mittel gegen entsprechende Alterungsvorgänge und therapeutischer Mittel gegen damit einhergehende Krankheiten, wobei man ein Naphthyridiumderivat gemäß Anspruch 1 als Indikator verwendet.

7. In vitro-Verfahren zur Bewertung der Wirkung von Pharmazeutika wie therapeutischer Mittel gegen Diabetes, solcher gegen diabetische Komplikationen, solcher gegen Dialyse-bezogene Komplikationen, vorbeugender Mittel gegen entsprechende Alterungsvorgänge und therapeutischer Mittel gegen damit einhergehende Krankheiten, wobei man einen Antikörper gemäß Anspruch 2 verwendet.

8. Verwendung eines Naphthyridiumderivats gemäß Anspruch 1 zur Herstellung einer Zubereitung zur Verwendung für die Diagnose von Diabetes, diabetischer Komplikationen, Dialyse-bezogener Komplikationen, entsprechender Alterungsabläufe und damit einhergehender Krankheiten.

9. Verwendung eines Antikörpers gemäß Anspruch 2 zur Herstellung einer Zubereitung zur Verwendung für die Diagnose von Diabetes, diabetischer Komplikationen, Dialyse-bezogener Komplikationen, entsprechender Alterungsabläufe und damit einhergehender Krankheiten.

10. Verwendung eines Naphthyridiumderivats gemäß Anspruch 1 zur Herstellung einer Zubereitung zur Verwendung für die Bewertung der Wirkung von Pharmazeutika wie therapeutischer Mittel gegen Diabetes, solcher gegen diabetische Komplikationen, solcher gegen Dialyse-bezogene Komplikationen, vorbeugender Mittel gegen entsprechende Alterungsabläufe und therapeutischer Mittel gegen damit einhergehende Krankheiten.

11. Verwendung eines Antikörpers gemäß Anspruch 2 zur Herstellung einer Zubereitung zur Verwendung für die Bewertung der Wirkung von Pharmazeutika wie therapeutischer Mittel gegen Diabetes, solcher gegen diabetische Komplikationen, solcher gegen Dialyse-bezogene Komplikationen, vorbeugender Mittel gegen entsprechende Alterungsabläufe und therapeutischer Mittel gegen damit einhergehende Krankheiten.

## Revendications

1. Dérivé naphtyridinium représenté par la formule générale (I) 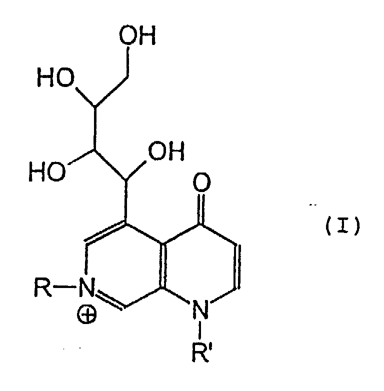 dans laquelle R et R' sont des groupes alkyle qui ont de 1 à 6 atomes de carbone, qui peuvent être les mêmes ou différents et qui peuvent être substitués avec un ou plusieurs groupes amine, carboxyle et/ou amine protégée, le groupe protecteur étant un groupe formyle, acétyle, tosyle, benzyloxycarbonyle, p-méthoxybenzyloxycarbonyle, p-chlorobenzyloxycarbonyle, p-nitrobenzyloxycarbonyle,
p-phénylazobenzyloxycarbonyle,
p-méthoxyphénylazobenzyloxycarbonyle, tert-butoxycarbonyle, tert-amyloxycarbonyle, p-biphénylisopropyloxycarbonyle et diisopropylméthyloxycarbonyle, ainsi que leurs sels.

2. Anticorps qui peut être obtenu en utilisant un dérivé naphtyridinium selon la revendication 1 comme haptène.

3. Utilisation d'un dérivé naphtyridinium selon la revendication 1 comme haptène pour la préparation d'un anticorps selon la revendication 2.

4. Méthode in vitro pour le diagnostic d'un diabète, de complications liées au diabète, de complications liées à une dialyse, d'un vieillissement et de maladies liées au vieillissement , faisant appel à un dérivé naphtyridinium selon la revendication 1 comme indicateur.

5. Méthode in vitro pour diagnostiquer un diabète, des complications liées au diabète, des complications liées à la dialyse, un vieillissement et des maladies liées au vieillissement, faisant appel à un anticorps selon la revendication 2.

6. Méthode in vitro pour évaluer l'effet de composés pharmaceutiques tels que les agents thérapeutiques contre le diabète, contre des complications liées au diabète, contre des complications liées à la dialyse, les agents de prévention du vieillissement et les agents thérapeutiques contre des maladies liées au vieillissement, faisant appel à un dérivé naphtyridinium selon la revendication 1 comme indicateur.

7. Méthode in vitro pour l'évaluation des effets de composés pharmaceutiques tels que les agents thérapeutiques contre le diabète, contre des complications liées au diabète, contre des complications liées à la dialyse, les agents de prévention du vieillissement et les agents thérapeutiques contre des maladies liées au vieillissement, faisant appel à un anticorps selon la revendication 2.

8. Utilisation d'un dérivé naphtyridinium selon la revendication 1 pour la production d'une préparation utile pour le diagnostic d'un diabète, de complications liées au diabète, de complications liées à la dialyse, d'un vieillissement et de maladies liées au vieillissement.

9. Utilisation d'un anticorps selon la revendication 2 pour la production d'une préparation utile pour le diagnostic d'un diabète, de complications liées au diabète, de complications liées à la dialyse, d'un vieillissement et de maladies liées au vieillissement.

10. Utilisation d'un dérivé naphtyridinium selon la revendication 1 pour la production d'une préparation utile pour l'évaluation de l'effet de composés pharmaceutiques tels que les agents thérapeutiques contre le diabète, contre des complications liées au diabète, contre des complications liées à la dialyse, les agents de prévention du vieillissement et les agents thérapeutiques contre des maladies liées au vieillissement.

11. Utilisation d'un anticorps selon la revendication 2, pour la production d'une préparation utile pour l'évaluation de l'effet de composés pharmaceutiques tels que les agents thérapeutiques contre le diabète, contre des complications liées au diabète, contre des complications liées à la dialyse, les agents de prévention du vieillissement et les agents thérapeutiques contre les maladies liées au vieillissement.
